# EUROPEAN PATENT APPLICATION

(11) **EP 4 624 469 A1**
(43) Date of publication of application: **01.10.2025**
(21) Application number: 23894025.8
(22) Date of filing: 24.11.2023
(51) Int. Cl.: C07D 405/12, A61K 31/47, A61K 31/4709, A61P 35/00, A61P 1/00, A61P 29/00

(54) **QUINOLINE AMINE COMPOUND CRYSTAL FORM AND PREPARATION METHOD THEREFOR**

(30) Priority: 25.11.2022 CN 202211491797
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: ZHAO, Xianghu, Shanghai 200245 (CN); YOU, Lingfeng, Shanghai 200245 (CN); FENG, Jun, Shanghai 200245 (CN); HE, Feng, Shanghai 200245 (CN); YANG, Junran, Lianyungang, Jiangsu 222047 (CN); DU, Zhenxing, Lianyungang, Jiangsu 222047 (CN)
(74) Representative: dompatent von Kreisler Selting Werner - Partnerschaft von Patent- und Rechtsanwälten mbB
(86) International application number: PCT/CN2023/134047
(87) International publication number: WO 2024/109936

(57) **Abstract**

The present disclosure relates to a quinoline amine compound crystal form and a preparation method therefor. Specifically, the present disclosure provides a crystal form of 8-chloro-*N*-(2,2-difluorobenzo[*d*][1,3]dioxolane-5-yl)quinoline-2-amine and a preparation method therefor. The corresponding crystal form has good stability and can be better utilized in clinical treatment.

## Description

The present application claims priority to Chinese Patent Application No. 2022114917970 filed on November 25, 2022, which is incorporated herein by reference in its entirety.

### TECHNICAL FIELD

The present disclosure pertains to the field of pharmaceutics and relates to a crystal form of a quinolinamine compound and a preparation method therefor.

### BACKGROUND

miR-124 is widely expressed in tissues throughout the body and is particularly highly expressed in brain tissues. Research shows that the overexpression of miR-124 can promote the transition of activated macrophages/microglia to quiescence, thereby inhibiting the autoimmune disease encephalomyelitis. In addition, miR-124 can promote the conversion of macrophages to M2 type, thereby exerting anti-inflammatory effects. miR-124 also affects T cell differentiation, and miR-124-treated T cells have decreased levels of both IFN-y and TNFα. The overexpression of miR-124 exerts anti-inflammatory effects by down-regulating STAT3 protein so as to reduce the expression of inflammatory cytokine IL-17 and inhibit the differentiation of Th17 cells. The above studies indicate that the development of a novel small-molecule drug for up-regulating miR-124 can be used to effectively treat related inflammatory diseases.

Related published patent applications include WO2010143169A2, WO2015001518A1, WO2016009065A2, WO2017158201A1, WO2020127843A1, etc.

WO2022247920 discloses a quinolinamine compound capable of up-regulating miR-124, the structure of which is as follows:

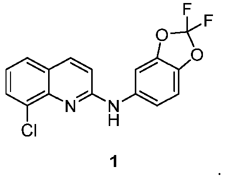

The structure of a crystal form used as a pharmaceutically active ingredient often affects the chemical and physical stabilities of the drug. Changes in crystallization and storage conditions may cause changes in the crystal structure of the compound and sometimes the generation of other crystal forms. Generally, drug products of amorphous forms do not have a regular crystal structure and often have other defects, such as having relatively poor product stability, being relatively difficult to filter, being prone to agglomeration, and having poor flowability. Therefore, the research on its crystal forms has important significance for the development of drugs that are suitable for industrial production and have good bioactivity.

### SUMMARY

The present disclosure provides, in one aspect, a crystal form A of the compound 8-chloro-*N*-(2,2-difluorobenzo[*d*][1,3]dioxol-5-yl)quinolin-2-amine, wherein an X-ray powder diffraction pattern expressed in terms of 2*θ* angles, which are diffraction angles, of the crystal form has characteristic peaks at 7.237, 9.232, 13.702, 14.459, and 18.917.

In some embodiments, the X-ray powder diffraction pattern expressed in terms of 2*θ* angles, which are diffraction angles, of the crystal form A has characteristic peaks at 7.237, 9.232, 13.702, 14.459, 18.917, 24.428, and 29.321.

In some embodiments, the X-ray powder diffraction pattern expressed in terms of 2*θ* angles, which are diffraction angles, of the crystal form A has characteristic peaks at 7.237, 9.232, 13.702, 14.459, 18.033, 18.917, 24.428, 25.521, and 29.321.

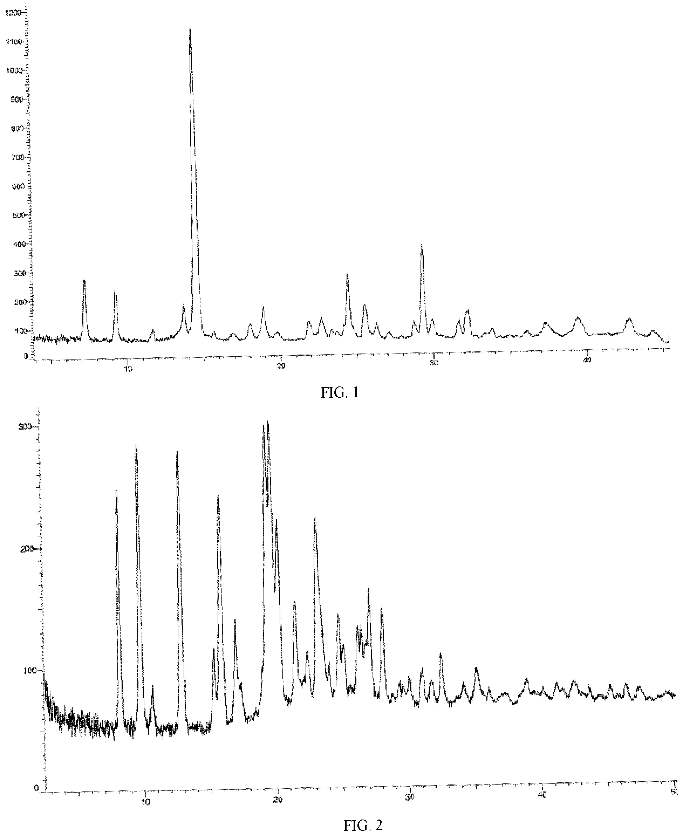

In some other embodiments, the X-ray powder diffraction pattern expressed in terms of 2*θ* angles, which are diffraction angles, of the crystal form A is shown in FIG. 1.

The present disclosure further provides a method for preparing the aforementioned crystal form A of the compound, wherein the method is selected from the group consisting of any one of the following methods:
method 1:
   (a) mixing the compound 8-chloro-*N*-(2,2-difluorobenzo[*d*][1,3]dioxol-5-yl)quinolin-2-amine with a solvent (1) and stirring to dissolve or heating to dissolve, and
   (b) adding a solvent (2) and crystallizing,
wherein the solvent (1) is selected from the group consisting of acetonitrile, methanol, ethanol, isopropanol, acetone, ethyl acetate, isopropyl acetate, tetrahydrofuran, methyl isopropyl ketone, dichloromethane, 10% water/methanol, 7% water/ethanol, 10% water/isopropanol, and 10% water/acetone, and the solvent (2) is selected from the group consisting of water, cyclohexane, and n-heptane;
and, method 2:
   (a) mixing the compound 8-chloro-*N*-(2,2-difluorobenzo[*d*][1,3]dioxol-5-yl)quinolin-2-amine with a solvent (3) and stirring to dissolve or heating to dissolve, and
   (b) crystallizing,
wherein the solvent (3) is selected from the group consisting of tetrahydrofuran, ethyl acetate, and dichloromethane;
and, method 3:
   (a) mixing the compound 8-chloro-*N*-(2,2-difluorobenzo[*d*][1,3]dioxol-5-yl)quinolin-2-amine with a solvent (4), and
   (b) slurrying by stirring, wherein the solvent (4) is selected from the group consisting of water, cyclohexane, n-heptane, methanol, ethanol, isopropanol, dichloromethane, 1,4-dioxane, 10% water/methanol, 7% water/ethanol, and 10% water/isopropanol.

The present disclosure provides, in one aspect, a crystal form B of the compound 8-chloro-*N*-(2,2-difluorobenzo[*d*][1,3]dioxol-5-yl)quinolin-2-amine, wherein an X-ray powder diffraction pattern expressed in terms of 2*θ* angles, which are diffraction angles, of the crystal form has characteristic peaks at 8.205, 9.781, 12.87, 15.907, and 19.796.

In some embodiments, the X-ray powder diffraction pattern expressed in terms of 2*θ* angles, which are diffraction angles, of the crystal form B has characteristic peaks at 8.205, 9.781, 12.870, 15.907, 19.448, 19.796, 20.264, and 23.185.

In some embodiments, the X-ray powder diffraction pattern expressed in terms of 2*θ* angles, which are diffraction angles, of the crystal form B has characteristic peaks at 8.205, 9.781, 10.672, 12.87, 15.356, 15.907, 16.997, 19.448, 19.796, 20.264, and 23.185.

In some other embodiments, the X-ray powder diffraction pattern expressed in terms of 2*θ* angles, which are diffraction angles, of the crystal form B is shown in FIG. 2.

The present disclosure further provides a method for preparing the aforementioned crystal form B of the compound, wherein the method comprises:
(a) mixing the compound 8-chloro-*N*-(2,2-difluorobenzo[*d*][1,3]dioxol-5-yl)quinolin-2-amine with a solvent (5) and stirring to dissolve or heating to dissolve, and
(b) adding a solvent (6) and crystallizing,
wherein the solvent (5) is selected from the group consisting of dimethyl sulfoxide, and the solvent (6) is selected from the group consisting of water.

The present disclosure provides, in one aspect, a crystal form C of the compound 8-chloro-*N*-(2,2-difluorobenzo[*d*][1,3]dioxol-5-yl)quinolin-2-amine, wherein an X-ray powder diffraction pattern expressed in terms of 2*θ* angles, which are diffraction angles, of the crystal form has characteristic peaks at 9.296, 15.522, 18.784, 23.216, and 25.889.

In some embodiments, the X-ray powder diffraction pattern expressed in terms of 2*θ* angles, which are diffraction angles, of the crystal form C has characteristic peaks at 7.753, 9.296, 15.522, 18.784, 21.398, 23.216, and 25.889.

In some embodiments, the X-ray powder diffraction pattern expressed in terms of 2*θ* angles, which are diffraction angles, of the crystal form C has characteristic peaks at 7.353, 7.753, 9.296, 14.475, 15.522, 16.248, 17.320, 18.784, 21.398, 23.216, and 25.889.

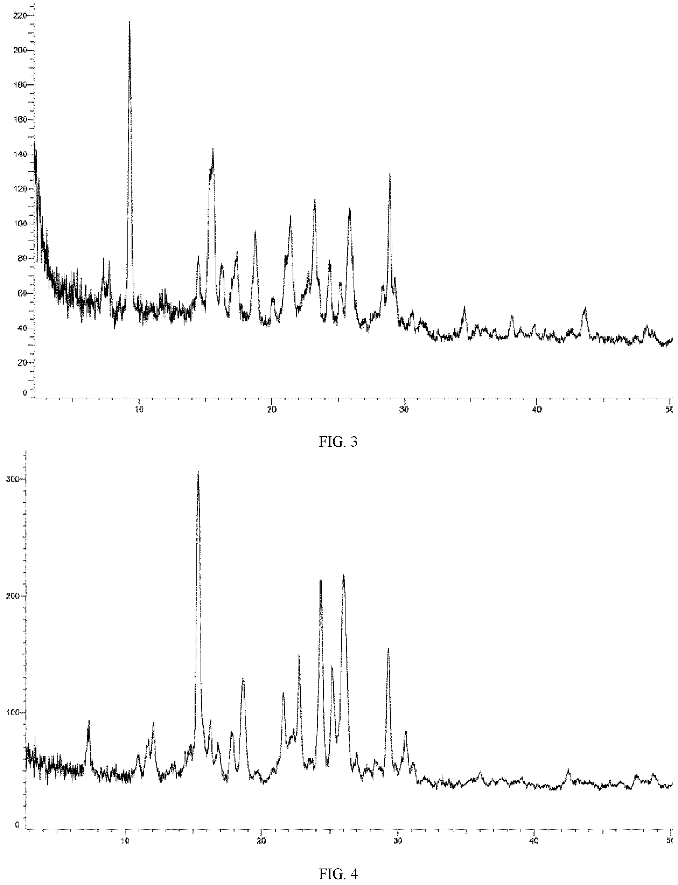

In some other embodiments, the X-ray powder diffraction pattern expressed in terms of 2*θ* angles, which are diffraction angles, of the crystal form C is shown in FIG. 3.

The present disclosure further provides a method for preparing the aforementioned crystal form C of the compound, wherein the method comprises:
(a) mixing the compound 8-chloro-*N*-(2,2-difluorobenzo[*d*][1,3]dioxol-5-yl)quinolin-2-amine with a solvent (7) and stirring to dissolve or heating to dissolve, and
(b) adding a solvent (8) and crystallizing,
wherein the solvent (7) is selected from the group consisting of 1,4-dioxane, and the solvent (8) is selected from the group consisting of n-heptane.

The present disclosure provides, in one aspect, a crystal form D of the compound 8-chloro-*N*-(2,2-difluorobenzo[*d*][1,3]dioxol-5-yl)quinolin-2-amine, wherein an X-ray powder diffraction pattern expressed in terms of 2*θ* angles, which are diffraction angles, of the crystal form has characteristic peaks at 7.350, 12.084, 15.384, 18.643, and 29.312.

In some embodiments, the X-ray powder diffraction pattern expressed in terms of 2*θ* angles, which are diffraction angles, of the crystal form D has characteristic peaks at 7.350, 12.084, 15.384, 16.260, 17.855, 18.643, 21.610, and 29.312.

In some embodiments, the X-ray powder diffraction pattern expressed in terms of 2*θ* angles, which are diffraction angles, of the crystal form D has characteristic peaks at 7.350, 12.084, 15.384, 16.260, 17.855, 18.643, 21.610, 22.768, 24.347, 25.201, 26.038, and 29.312.

In some other embodiments, the X-ray powder diffraction pattern expressed in terms of 2*θ* angles, which are diffraction angles, of the crystal form D is shown in FIG. 4.

The present disclosure further provides a method for preparing the aforementioned crystal form D of the compound, wherein the method comprises:
(a) mixing the compound 8-chloro-*N*-(2,2-difluorobenzo[*d*][1,3]dioxol-5-yl)quinolin-2-amine with a solvent (9) and stirring to dissolve or heating to dissolve, and
(b) adding a solvent (10) and crystallizing,
wherein the solvent (9) is selected from the group consisting of tetrahydrofuran, and the solvent (10) is selected from the group consisting of water.

The present disclosure provides, in one aspect, a crystal form E of the compound 8-chloro-*N*-(2,2-difluorobenzo[*d*][1,3]dioxol-5-yl)quinolin-2-amine, wherein an X-ray powder diffraction pattern expressed in terms of 2*θ* angles, which are diffraction angles, of the crystal form has characteristic peaks at 13.489, 18.000, 23.559, 24.276, and 26.328.

In some embodiments, the X-ray powder diffraction pattern expressed in terms of 2*θ* angles, which are diffraction angles, of the crystal form E has characteristic peaks at 13.489, 16.863, 18.000, 23.559, 24.276, 26.108, 26.328, and 27.094.

In some embodiments, the X-ray powder diffraction pattern expressed in terms of 2*θ* angles, which are diffraction angles, of the crystal form E has characteristic peaks at 13.489, 14.587, 16.863, 18.000, 18.943, 23.279, 23.559, 24.276, 25.768, 26.108, 26.328, and 27.094.

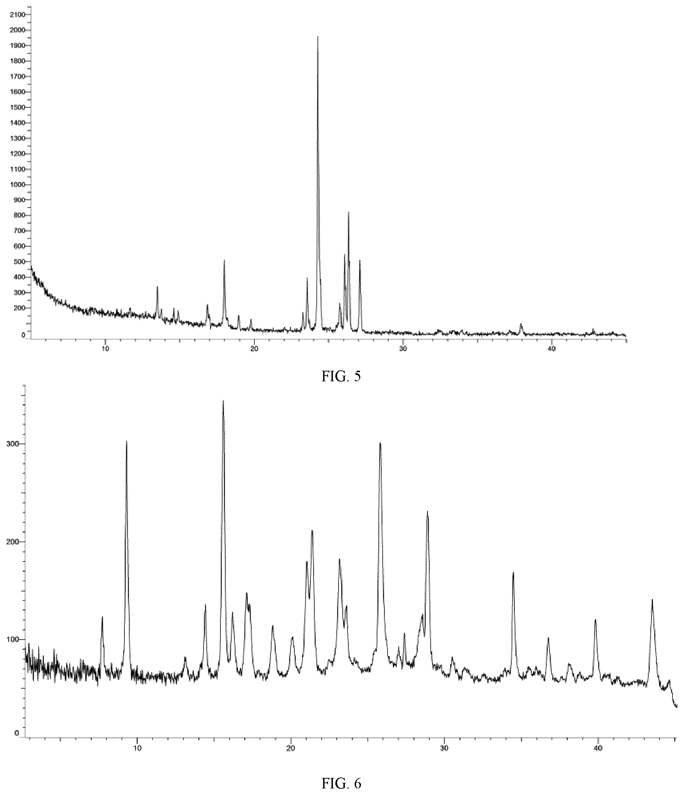

In some other embodiments, the X-ray powder diffraction pattern expressed in terms of 2*θ* angles, which are diffraction angles, of the crystal form E is shown in FIG. 5.

The present disclosure provides, in one aspect, a crystal form F of the compound 8-chloro-*N*-(2,2-difluorobenzo[*d*][1,3]dioxol-5-yl)quinolin-2-amine, wherein an X-ray powder diffraction pattern expressed in terms of 2*θ* angles, which are diffraction angles, of the crystal form has characteristic peaks at 9.299, 14.439, 15.621, 16.200, and 17.314.

In some embodiments, the X-ray powder diffraction pattern expressed in terms of 2*θ* angles, which are diffraction angles, of the crystal form F has characteristic peaks at 7.734, 9.299, 14.439, 15.621, 16.200, 17.314, 21.395, and 25.814.

In some embodiments, the X-ray powder diffraction pattern expressed in terms of 2*θ* angles, which are diffraction angles, of the crystal form F has characteristic peaks at 7.734, 9.299, 14.439, 15.621, 16.200, 17.314, 21.055, 21.395, 23.194, 25.814, 28.906, 34.485, and 43.544.

In some other embodiments, the X-ray powder diffraction pattern expressed in terms of 2*θ* angles, which are diffraction angles, of the crystal form F is shown in FIG. 6.

Further, in the X-ray powder diffraction pattern expressed in terms of 2*θ* angles, which are diffraction angles, of the crystal form A, crystal form B, crystal form C, crystal form D, crystal form E, or crystal form F of the compound of the present disclosure, the 2*θ* angles have a margin of error of ±0.2.

In certain embodiments, the preparation methods for the crystal forms of the present disclosure further comprise any one of a filtration step, a washing step, or a drying step.

In some embodiments, the crystallization includes, but is not limited to, stirring crystallization (antisolvent crystallization and slurrying crystallization) and volatilizing crystallization.

In some embodiments, the drying includes, but is not limited to, blow-drying and vacuum drying. Generally, the drying temperature is 25 °C-100 °C, preferably 30 °C-70 °C, such as 40 °C, 50 °C, or 60 °C.

In another aspect, the present disclosure further provides a pharmaceutical composition comprising an aforementioned crystal form and a pharmaceutically acceptable excipient.

The present disclosure further provides a pharmaceutical composition prepared from an aforementioned crystal form and a pharmaceutically acceptable excipient.

The present disclosure further provides a preparation method for a pharmaceutical composition, the method comprising a step of mixing an aforementioned crystal form with a pharmaceutically acceptable excipient.

The present disclosure further provides use of the aforementioned crystal forms or pharmaceutical composition in the preparation of a medicament for regulating miRNA levels, wherein preferably, the miRNA is miR-124. The present disclosure further provides use of the aforementioned crystal forms or pharmaceutical composition in a medicament for treating and/or preventing a disease or condition, wherein the disease or condition is selected from the group consisting of an inflammation and a cancer.

In some embodiments, the inflammation is inflammatory bowel disease. In some embodiments, the cancer is melanoma or breast cancer.

As used herein, "2*θ* or 2*θ* angle" refers to a diffraction angle; *θ* refers to the Bragg angle in ° or degrees; the 2*θ* of each characteristic peak has a margin of error of ±0.20 (including the case that a number having more than 1 decimal place is rounded), specifically -0.20, -0.19, -0.18, -0.17, -0.16, -0.15, -0.14, -0.13, -0.12, -0.11, -0.10, -0.09, -0.08, -0.07, -0.06, -0.05, -0.04, -0.03, -0.02, -0.01, 0.00, 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, or 0.20.

As used herein, "differential scanning calorimetry" or "DSC" refers to the measurement of the temperature difference and heat flow difference between a sample and a reference substance during a ramping or thermostatic process to characterize all the physical changes and chemical changes related to the thermal effect, and to obtain phase change information about the sample.

In the present disclosure, the drying temperature is generally 25 °C-100 °C, preferably 30 °C-70 °C, and the drying may be performed under atmospheric pressure or reduced pressure.

As used herein, "pharmaceutically acceptable excipient" includes, but is not limited to, any auxiliary, carrier, glidant, sweetener, diluent, preservative, dye/colorant, flavoring agent, surfactant, wetting agent, dispersant, suspending agent, stabilizer, isotonic agent, or emulsifier that has been approved by the U.S. Food and Drug Administration as acceptable for use in humans or livestock animals.

As used herein, "slurrying" or "slurried" refers to a purification method utilizing the low solubility of the target substance and high solubility of the impurities in a solvent, which is capable of decoloring, changing the crystal form, or removing small amounts of impurities.

The crystal forms of the present disclosure include, but are not limited to, solvates of the compound represented by formula (I), wherein the solvents include, but are not limited to, water.

### BRIEF DESCRIPTION OF THE DRAWINGS

FIG. 1 shows an XRPD pattern of the crystal form A of compound 1.
FIG. 2 shows an XRPD pattern of the crystal form B of compound 1.
FIG. 3 shows an XRPD pattern of the crystal form C of compound 1.
FIG. 4 shows an XRPD pattern of the crystal form D of compound 1.
FIG. 5 shows an XRPD pattern of the crystal form E of compound 1.
FIG. 6 shows an XRPD pattern of the crystal form F of compound 1.

### DETAILED DESCRIPTION

The present disclosure is further illustrated in detail by the following examples and experimental examples.

These examples and experimental examples are illustrative only and are not intended to limit the scope of the present disclosure.

Test conditions for the instruments used in the experiments:
The structures of the compounds were determined by nuclear magnetic resonance (NMR) spectroscopy or/and mass spectrometry (MS). The NMR shifts (δ) are given in 10⁻⁶ (ppm). The NMR analyses were performed using a Bruker AVANCE-400 nuclear magnetic resonance system, with dimethyl sulfoxide-D6 (DMSO-*d*₆), chloroform-D (CDCl₃), and methanol-D4 (CD₃OD) as solvents and tetramethylsilane (TMS) as an internal standard.

The MS analyses were performed using an Agilent 1200/1290 DAD-6110/6120 Quadrupole MS liquid chromatography-mass spectrometry system (manufacturer: Agilent; MS model: 6110/6120 Quadrupole MS), waters ACQuity UPLC-QD/SQD (manufacturer: waters; MS model: waters ACQuity Qda Detector/waters SQ Detector), and THERMO Ultimate 3000-Q Exactive (manufacturer: THERMO; MS model: THERMO Q 15 Exactive).

The HPLC analyses were performed using an Agilent 1260DAD high pressure liquid chromatograph (Sunfire C18 150 × 4.6 mm chromatography column) and a Thermo U3000 high pressure liquid chromatograph (Gimini C18 150 × 4.6 mm chromatography column).

XRPD refers to X-ray powder diffraction: The analysis was performed using a BRUKER D8 X-ray diffractometer; specific acquisition information: a Cu anode (40 kV, 40 mA), ray: monochromatic Cu-Ka ray (l = 1.5418 Å). Scan mode: *θ*/2*θ*; scan range: 3-48°.

DSC refers to differential scanning calorimetry: The measurement was performed using a METTLER TOLEDO DSC 3+ differential scanning calorimeter, with a temperature ramp rate of 10 °C/min, 25-300 °C or 25-350 °C, and a nitrogen purge speed of 50 mL/min.

TGA refers to thermogravimetric analysis: The analysis was performed using a METTLER TOLEDO TGA 2 thermogravimetric analyzer, with a temperature ramp rate of 10 °C/min, specific temperature ranges shown in corresponding patterns, and a nitrogen purge speed of 50 mL/min.

DVS refers to dynamic vapor sorption: Surface Measurement Systems instrinsic was adopted; the humidity started at 50%, the humidity range of the investigation was 0%-95%, and the humidity was increased in increments of 10%; the criterion was that each gradient mass change dM/dT is ≤ 0.002%, TMAX 360 min, two cycles.

The known starting materials in the present disclosure may be synthesized by using or according to methods known in the art, or may be purchased from companies such as ABCR GmbH & Co. KG, Acros Organics, Aldrich Chemical Company, Accela ChemBio Inc., and Chembee Chemicals.

The monitoring of the reaction progress in the examples was performed by thin-layer chromatography (TLC). The developing solvents for the reactions, the eluent systems for the column chromatography purification, and the developing solvent systems for the thin-layer chromatography include: A: a dichloromethane/methanol system, and B: a n-hexane/ethyl acetate system. The volume ratio of the solvents was adjusted depending on the polarity of the compound, or small amounts of basic or acidic reagents such as triethylamine and acetic acid may also be added for adjustments.

### Example 1. Synthesis of 8-Chloro-N-(2,2-difluorobenzo[d][1,3]dioxol-5-yl)quinolin-2-amine (with reference to the preparation method of Example 1 in Application No. WO2022247920)

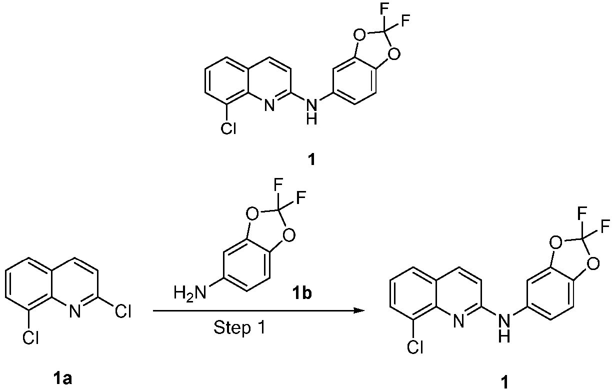

2,8-Dichloroquinoline 1a (100 mg, 0.51 mmol, Bide Pharmatech) and 5-amino-2,2-difluoro-1,3-benzo[1,3]dioxolane 1b (105 mg, 0.61 mmol, Shanghai Haohong) were dissolved in isopropanol (1 mL), and the solution was heated to 90 °C and left to react for 12 h. The reaction mixture was filtered and then purified by preparative high performance liquid chromatography (Waters 2767-SQ Detecor2, elution system: a 0.1% aqueous formic acid solution and acetonitrile, acetonitrile gradient: 65%-85%, flow rate: 30 mL/min) to give the title compound 1 (150 mg, yield: 89.0%).

MS m/z (ESI): 335.0 [M+1].

¹H NMR (500 MHz, DMSO-*d*₆) δ 9.99 (s, 1H), 8.88 (d, 1H), 8.17 (d, 1H), 7.81 (dd, 1H), 7.77 (dd, 1H), 7.50 (dd, 1H), 7.39 (d, 1H), 7.32 (t, 1H), 7.14 (d, 1H).

### Test Example 1. Up-Regulation of miR-124

### I. Experimental materials and instruments

1. Dynabead Human T-Activator CD3/CD28 for T Cell Expansion and Activation (Gibco, 11131D)
2. Pan T Cell Isolation Kit, human (Miltenyi, 130-096-535)
3. Human IL-2 (Peprotech, 200-02-100)
4. microRNA extraction kit (Qiagen, 217004)
5. miScript II RT Kit (Qiagen, 218161)
6. miScript SYBR Green PCR Kit (Qiagen, 218073)
7. Phosphate-buffered saline (PBS), pH 7.4 (Shanghai BasalMedia Technologies Co., Ltd., B320)
8. Bovine serum albumin (BSA) (Beyotime, ST023)
9. EDTA (0.5 M), pH 8.0 (Invitrogen, AM9260G)
10. LS Columns (Miltenyi, 130-042-401)
11. 24-well cell culture plates (Corning, 3524)
12. 96-well plates (Corning, 3788)
13. Cell incubator (Thermo, Steri cycle i160)
14. Real-time fluorescence quantitative PCR system (Applied biosystem, QuantStudio6 Flex)
15. PCR system (Applied biosystem, ProFlex)
16. 96-well clear PCR plate, 0.2 mL (Applied biosystems, N8010560)
17. RPMI1640 culture medium (Gibco, 11875119)
18. Fetal bovine serum (FBS) (Gibco, 10099-141)
19. Magnetic rack (Invitrogen, DynaMag^{™}-2)
20. Six-well cell culture plates (Thermo, 150239)
21. Spectrophotometer (IMPLEN, NP80)
22. QuadroMACS Separator (Miltenyi, 130-090-976)
23. miR124-3P-F primer (customized by Genewiz)
24. hsa-U6 detection primer (Tiangen, CD201-0145)

### II. Experimental steps

The effect of the compound on miR-124 expression levels was assessed in CD3/CD28 antibody-activated T cells. After the activated T cells were treated with the compound, the total cellular RNA was extracted, the cDNA obtained from reverse transcription was used as a template, and quantification was performed by using an SYBR green fluorescence quantitative PCR method and a specific miR-124 primer.

Isolation of T cells: The purchased human peripheral blood mononuclear cells (PBMCs) were counted, filtered, and then washed once with a separation buffer (PBS, pH 7.4, containing 0.5% BSA and 2 mM EDTA), and the supernatant was discarded. Various components were added in such a manner that for every 1 × 10⁷ cells, 40 µL of buffer and 10 µL of pan T Cell Biotin-Antibody Cocktail were added. The pellets were resuspended and well mixed, and the suspension was incubated in a refrigerator at 4 °C for 5 min. After the incubation was completed, various components were added in such a manner that for every 1 × 10⁷ cells, 30 µL of buffer and 20 µL of Pan T Cell MicroBeads Cocktail were added. After thorough mixing, the mixture was incubated in a refrigerator at 4 °C for 10 min. The separation column (LS column) was rinsed with 3 mL of cell separation buffer in advance, and the above cell suspension was loaded onto the column. After the cell suspension passed through the column, the column was repeatedly washed 3 times with 1 mL of cell separation buffer, and the cell eluate, which contained enriched T cells, was collected in a 15 mL centrifuge tube. The cells were counted and added at a density of 1 × 10⁶ cells/mL to an RPMI1640 culture medium (complete culture medium) containing 10% FBS and 40 U/mL IL-2, and the mixture was stored on ice for later use.

T cell activation: T cell activation CD3/CD28 magnetic beads were added to a 1.5 mL centrifuge tube in such a manner that for every 1 × 10⁶ cells, 25 µL of activation magnetic beads was added. Before pipetting, the magnetic beads should be shaken on a shaker for about 30 s. In the centrifuge tube, the activation magnetic beads were washed 3 times with culture medium, in a ratio of greater than 1:1 by volume. After the last wash, all of the washing liquid was removed, and an amount of complete culture medium that was equal to the starting volume was added to resuspend the activation magnetic beads. The washed activation magnetic beads were added to the cell resuspension and well mixed. The cells were added to a six-well plate at 3 mL/well, and the plate was incubated in a 37 °C, 5% CO₂ cell incubator for 2 days.

Compound treatment: A 20 mM compound stock solution was diluted to 200 µM with DMSO and then diluted 4-fold to 50 µM (50×) with complete culture medium, and the solution was well mixed for later use. Negative control wells were set using 4-fold diluted DMSO (25% DMSO). The T cells that had been activated for two days were well pipetted. The 1.5 mL centrifuge tube was mounted on a magnetic rack. The activation magnetic beads were removed, and the cell suspension was collected. After cell counting, the cell suspension was centrifuged at 300× g for 10 min, and the supernatant was discarded. The cells were resuspended at 1.02 × 10⁶ cells/mL. To each 24-well plate, 980 µL of cell suspension and 20 µL of 50× compound were added, with a final compound concentration of 1 µM. The cells were incubated in a 37 °C, 5% CO₂ cell incubator for another 3 days.

RNA extraction: T cells were collected by filtration, centrifuged at 1500 rpm for 3 min, washed once with PBS, and centrifuged, and the supernatant was then discarded. The total cellular RNA was extracted using a microRNA extraction kit according to the instructions. 700 µL of Trizol cell lysis buffer was added to cell pellets, and the mixture was well pipetted and left to stand at room temperature for 5 min. 140 µL of chloroform was added, and the mixture was well shaken and left to stand at room temperature for 3 min. The chloroform-cell lysis buffer mixture was centrifuged at 12000× g and 4 °C for 15 min. The supernatant was transferred to a new RNase-free centrifuge tube, and 1.5 volumes of absolute ethanol were added. The solution was pipetted several times, transferred to an RNA adsorption column, and centrifuged at 8000× g for 15 s. The centrifuge column was washed once with 700 µL of RWT solution, followed by 15 s of centrifugation at 8000× g. The column was washed twice with 500 µL of RPE solution, followed by 2 min of centrifugation at 8000× g. The adsorption column was placed in a new 2 mL centrifuge tube, and centrifugation was performed at 12,000× g for 1 min to remove the washing liquid residue. The adsorption column was placed in a new 1.5 mL centrifuge tube, and 30-50 µL of RNase-free water was added. After 2 min of centrifugation at 12,000× g, a solution was collected; the collected solution was an RNA solution. The RNA concentration was measured using a spectrophotometer. The RNA solution was stored in a freezer at -80 °C.

Reverse transcription: The above extracted RNA template was placed on ice. A miScript II RT Kit was taken out, and some of the components (containing 5× miScript HiSpec Buffer, 10× miScript nucleics Mix, and RNase-free water) were thawed at room temperature. The miScript Reverse Transcriptase mix component was thawed on ice. The components of each reaction (10 µL) were: 5× miScript HiSpec Buffer (2 µL), 10× miScript nucleics Mix (1 µL), miScript Reverse Transcriptase mix (1 µL), RNase-free water (2 µL), and RNA template (4 µL). The above reaction was prepared on ice. A sample was placed in a PCR system, and a program was set as follows: 37 °C for 60 min; 95 °C for 5 min; stored at 4 °C. The sample obtained after the completion of the reaction was a cDNA sample.

Fluorescence quantitative PCR: The transcription level of miR-124 was determined using an SYBR green staining method, and meanwhile the transcription level of housekeeping gene U6 was determined as an internal reference. All reagents required for the miScript SYBR Green PCR Kit were thawed to normal temperature, and each cDNA sample template was diluted 10-fold with RNase-free water and then diluted 5-fold. A reaction mixture was prepared according to Table 1 below and added to a 96-well PCR plate. The plate was sealed with a plate sealing film, and centrifugation was performed. The PCR reaction was performed on a fluorescence quantitative PCR system according to the steps in Table 2.

**Table 1. The components of the fluorescence quantitative PCR reaction**

| Component | miR-124 detection (µL) | U6 housekeeping gene detection (µL) |
|---|---|---|
| 2×QuantiTest SYBR Green PCR Master Mix | 12.5 | 12.5 |
| 10× miScript universal primer | 2.5 | 2.5 |
| 10× forward primer (5 µM) | 2.5 (miR124-3p-F) | 2.5 (Hsa-U6 primer) |
| RNase-free water | 5 | 5 |
| cDNA template | 2.5 (diluted 10-fold) | 2.5 (diluted 50-fold) |
| Total volume | 25 | 25 |

**Table 2. Fluorescence quantitative PCR steps**

| Step | | Temperature | Time | Number of cycles |
|---|---|---|---|---|
| Initial | | 95 °C | 15 min | 1 |
| 3-step cycle | Denaturation | 94 °C | 15 s | 40 |
| | Annealing | 55 °C | 30 s | |
| | Extension | 70 °C | 30 s | |

**Table 3. Fluorescence quantitative PCR detection primers**

| Primer name | Sequence (5'-3') | Source |
|---|---|---|
| miR124-3p-F | TAAGGCACGCGGTGAATGCC | Customized by Genewiz |
| Hsa-U6 primer | / | miRNA primer library of Tiangen Biotech Co., Ltd. |
| 10× miScript universal primer | / | Included in miScript SYBR Green PCR Kit |

Data analysis: Based on the CT values calculated by software, the ratio of the expression level of miR-124 to the expression level of the internal reference U6 was calculated for each sample, i.e., ΔCT (test compound) = CT_{miRNA-124} (test compound) - CT_{U6} (test compound). The relative expression level was calculated by the formula: relative expression level (test compound) = 2^{(-[ΔCT(test compound) - ΔCT(DMSO)])}.

Compound 1 up-regulated 3.9-fold miR-124, suggesting good activity in promoting miR124 up-regulation.

### Example 2: Preparation of Crystal Form A

250 mg of compound 1 was added to 2.5 mL of acetonitrile and dissolved by stirring, and 17.5 mL of water was added. The solution was stirred for crystallization. After 4 days of slurrying at room temperature, the mixture was filtered, and drying was performed *in vacuo* to give a solid.

According to X-ray powder diffraction analysis, the product was defined as crystal form A. Its X-ray powder diffraction data are shown in Table 4, and its X-ray powder diffraction pattern is shown in FIG. 1.

Its DSC thermogram showed an endothermic peak at 177.14 °C.

Its TGA thermogram showed a weight loss of 0.22% from 30 °C to 100 °C.

DVS testing showed that the sample had a hygroscopic weight gain of about 0.02% under normal storage conditions (i.e., room temperature and 60% RH), a hygroscopic weight gain of about 0.02% under accelerated experimental conditions (i.e., 70% RH), and a hygroscopic weight gain of about 0.18% under extreme conditions (i.e., 90% RH). In addition, after DVS testing, the crystal form was re-identified, and the result showed that the crystal form did not change.

**Table 4**

| Peak No. | 2θ [° or degrees] | d [Å] | Relative intensity (%) |
|---|---|---|---|
| 1 | 7.237 | 12.20503 | 19.4 |
| 2 | 9.232 | 9.57135 | 15.4 |
| 3 | 11.669 | 7.57727 | 3.5 |
| 4 | 13.702 | 6.45751 | 10.6 |
| 5 | 14.459 | 6.12095 | 100.0 |
| 6 | 15.636 | 5.66284 | 2.1 |
| 7 | 16.886 | 5.24623 | 1.5 |
| 8 | 18.033 | 4.91507 | 4.3 |
| 9 | 18.917 | 4.68753 | 9.3 |
| 10 | 19.769 | 4.48734 | 1.4 |
| 11 | 21.850 | 4.06431 | 4.9 |
| 12 | 22.674 | 3.91850 | 5.7 |
| 13 | 23.357 | 3.80552 | 1.7 |
| 14 | 23.676 | 3.75489 | 0.6 |
| 15 | 24.140 | 3.68383 | 3.3 |
| 16 | 24.428 | 3.64096 | 19.7 |
| 17 | 25.521 | 3.48749 | 9.8 |
| 18 | 26.287 | 3.38754 | 4.0 |
| 19 | 27.084 | 3.28970 | 1.3 |
| 20 | 28.720 | 3.10583 | 4.9 |
| 21 | 29.321 | 3.04353 | 29.5 |
| 22 | 29.920 | 2.98394 | 5.0 |
| 23 | 31.672 | 2.82283 | 5.5 |
| 24 | 32.197 | 2.77798 | 8.0 |
| 25 | 33.817 | 2.64849 | 2.4 |
| 26 | 36.097 | 2.48625 | 1.2 |
| 27 | 37.347 | 2.40584 | 3.3 |
| 28 | 39.428 | 2.28355 | 5.4 |
| 29 | 42.781 | 2.11199 | 5.6 |
| 30 | 44.327 | 2.04188 | 2.3 |

### Example 3: Preparation of Crystal Form A

Compound 1 (6 mg, 17.63 µmol) was dissolved in 0.3 mL of tetrahydrofuran. After the compound was completely dissolved, slow volatilization was performed to give a solid, and the solid was dried *in vacuo* at 45 °C for 3 h to give a product.

The product was identified as the crystal form A by X-ray powder diffraction.

### Example 4: Preparation of Crystal Form A

Compound 1 (11 mg, 32.87 µmol) was dissolved in 0.4 mL of ethyl acetate. After the compound was completely dissolved, slow volatilization was performed to give a solid, and the solid was dried *in vacuo* at 45 °C for 3 h to give a product.

The product was identified as the crystal form A by X-ray powder diffraction.

### Example 5: Preparation of Crystal Form A

Compound 1 (14 mg, 41.83 µmol) was dissolved in 0.5 mL of dichloromethane. After the compound was completely dissolved, slow volatilization was performed to give a solid, and the solid was dried *in vacuo* at 45 °C for 3 h to give a product.

The product was identified as the crystal form A by X-ray powder diffraction.

### Example 6: Preparation of Crystal Form A

Compound 1 (1.70 g, 5.08 mmol) was dispersed in 35 mL of cyclohexane and slurried by stirring for 48 h. The mixture was filtered, and the filter cake was collected and dried *in vacuo* at 45 °C for 16 h to give a product. The product was identified as the crystal form A by X-ray powder diffraction.

### Example 7: Preparation of Crystal Form A

10 mg of compound 1 was weighed out, added to 1 mL of purified water, and slurried at room temperature for 3 days. After filtration, the solid was dried *in vacuo* to give a product.

The product was identified as the crystal form A by X-ray powder diffraction.

### Example 8: Preparation of Crystal Form A

Reference was made to the method of Example 7. 10 mg of compound 1 was weighed out, added to a solvent, and slurried. After filtration, the solid was dried *in vacuo* to give a product.

The product was identified as the crystal form A by X-ray powder diffraction. The data are shown in Table 5.

**Table 5**

| | Solvent | Crystal form |
|---|---|---|
| 1 | 1 mL of n-heptane | Crystal form A |
| 2 | 1 mL of n-hexane | Crystal form A |
| 3 | 0.1 mL of methanol | Crystal form A |
| 4 | 0.1 mL of ethanol | Crystal form A |
| 5 | 0.1 mL of isopropanol | Crystal form A |
| 6 | 0.1 mL of dichloromethane | Crystal form A |
| 7 | 0.1 mL of 1,4-dioxane | Crystal form A |
| 8 | 0.1 mL of water/methanol (10%) | Crystal form A |
| 9 | 0.1 mL of water/ethanol (7%) | Crystal form A |
| 10 | 0.1 mL of water/isopropanol (10%) | Crystal form A |

### Example 9: Preparation of Crystal Form A

10 mg of compound 1 was weighed out, added to 0.2 mL of methanol, and completely dissolved by stirring at room temperature, and 0.2 mL of purified water was added. The solution was stirred for crystallization, and slurrying was performed at room temperature for 1 day. After filtration, the solid was dried *in vacuo* to give a product.

The product was identified as the crystal form A by X-ray powder diffraction.

### Example 10: Preparation of Crystal Form A

10 mg of compound 1 was weighed out, added to 0.2 mL of ethanol, and completely dissolved by stirring at room temperature, and 0.4 mL of n-heptane was added. The solution was stirred for 1 day, and volatilization was performed for crystallization to give a product.

The product was identified as the crystal form A by X-ray powder diffraction.

### Example 11: Preparation of Crystal Form A

10 mg of compound 1 was weighed out, added to 0.6 mL of isopropanol, and completely dissolved by stirring at room temperature, and 0.4 mL of n-heptane was added. The solution was stirred for 1 day, and volatilization was performed for crystallization to give a product.

The product was identified as the crystal form A by X-ray powder diffraction.

### Example 12: Preparation of Crystal Form A

10 mg of compound 1 was weighed out, added to 0.1 mL of acetone, and completely dissolved by stirring at room temperature, and 0.4 mL of n-heptane was added. The solution was stirred for 1 day, and volatilization was performed for crystallization to give a product.

The product was identified as the crystal form A by X-ray powder diffraction.

### Example 13: Preparation of Crystal Form A

10 mg of compound 1 was weighed out, added to 0.1 mL of ethyl acetate, and completely dissolved by stirring at room temperature, and 0.4 mL of n-heptane was added. The solution was stirred for 1 day, and volatilization was performed for crystallization to give a product.

The product was identified as the crystal form A by X-ray powder diffraction.

### Example 14: Preparation of Crystal Form A

10 mg of compound 1 was weighed out, added to 0.1 mL of isopropyl acetate, and completely dissolved by stirring at room temperature, and 0.4 mL of n-heptane was added. The solution was stirred for crystallization, and slurrying was performed at room temperature for 1 day. An additional 0.2 mL of n-heptane was added, and slurrying was performed for another 3 h. After filtration, the solid was dried *in vacuo* to give a product. The product was identified as the crystal form A by X-ray powder diffraction.

### Example 15: Preparation of Crystal Form A

10 mg of compound 1 was weighed out, added to 0.1 mL of tetrahydrofuran, and completely dissolved by stirring at room temperature, and 0.4 mL of n-heptane was added. The solution was stirred for 1 day, and volatilization was performed for crystallization to give a product.

The product was identified as the crystal form A by X-ray powder diffraction.

### Example 16: Preparation of Crystal Form A

10 mg of compound 1 was weighed out, added to 0.1 mL of methyl isopropyl ketone, and completely dissolved by stirring at room temperature, and 0.4 mL of n-heptane was added. The solution was stirred for 1 day, and volatilization was performed for crystallization to give a product.

The product was identified as the crystal form A by X-ray powder diffraction.

### Example 17: Preparation of Crystal Form A

10 mg of compound 1 was weighed out, added to 0.4 mL of dichloromethane, and completely dissolved by stirring at room temperature, and 0.4 mL of n-heptane was added for crystallization. Slurrying was performed at room temperature for 1 day. An additional 0.2 mL of n-heptane was added, and slurrying was performed for 3 h. After filtration, the solid was dried *in vacuo* to give a product.

The product was identified as the crystal form A by X-ray powder diffraction.

### Example 18: Preparation of Crystal Form A

10 mg of compound 1 was weighed out, added to 1 mL of 10% water/methanol, and completely dissolved by stirring at room temperature, and 0.1 mL of water was added. The solution was stirred for crystallization. An additional 0.1 mL of water was added, and slurrying was then performed for 1 day. After filtration, the solid was dried *in vacuo* to give a product.

The product was identified as the crystal form A by X-ray powder diffraction.

### Example 19: Preparation of Crystal Form A

10 mg of compound 1 was weighed out, added to 0.6 mL of 7% water/ethanol, and completely dissolved by stirring at room temperature, and 0.4 mL of n-heptane was added. Volatilization was performed for crystallization to give a product.

The product was identified as the crystal form A by X-ray powder diffraction.

### Example 20: Preparation of Crystal Form A

10 mg of compound 1 was weighed out, added to 0.6 mL of 10% water/isopropanol, and completely dissolved by stirring at room temperature, and 0.4 mL of n-heptane was added. Volatilization was performed for crystallization to give a product.

The product was identified as the crystal form A by X-ray powder diffraction.

### Example 21: Preparation of Crystal Form A

10 mg of compound 1 was weighed out, added to 0.1 mL of 10% water/acetone, and completely dissolved by stirring at room temperature, and 0.4 mL of n-heptane was added. Volatilization was performed for crystallization to give a product.

The product was identified as the crystal form A by X-ray powder diffraction.

### Example 22: Preparation of Crystal Form A

10 mg of compound 1 was weighed out, added to 0.2 mL of ethanol, and dissolved by stirring at room temperature, and 0.1 mL of purified water was added. The solution was stirred for precipitation. Another 0.2 mL of purified water was added, and the mixture was then stirred for 1 day. After filtration, the solid was dried *in vacuo* to give a product.

The product was identified as the crystal form A by X-ray powder diffraction.

### Example 23: Preparation of Crystal Form A

10 mg of compound 1 was weighed out, added to 0.2 mL of ethanol, and dissolved by stirring at room temperature, and 0.4 mL of cyclohexane was added. After 1 day of stirring, volatilization was performed for crystallization to give a product.

The product was identified as the crystal form A by X-ray powder diffraction.

### Example 24: Preparation of Crystal Form A

10 mg of compound 1 was weighed out, added to 0.1 mL of acetone, and dissolved by stirring at room temperature, and 0.1 mL of purified water was added. The solution was stirred for precipitation. Another 0.2 mL of purified water was added, and the mixture was then stirred for 1 day. After filtration, the solid was dried *in vacuo* to give a product.

The product was identified as the crystal form A by X-ray powder diffraction.

### Example 25: Preparation of Crystal Form A

10 mg of compound 1 was weighed out, added to 0.1 mL of acetone, and dissolved by stirring at room temperature, and 0.4 mL of cyclohexane was added. After 1 day of stirring, volatilization was performed for crystallization to give a product.

The product was identified as the crystal form A by X-ray powder diffraction.

### Example 26: Preparation of Crystal Form A

10 mg of compound 1 was weighed out, added to 0.1 mL of ethyl acetate, and dissolved by stirring at room temperature, and 0.4 mL of cyclohexane was added. After 1 day of stirring, volatilization was performed for crystallization to give a product.

The product was identified as the crystal form A by X-ray powder diffraction.

### Example 27: Preparation of Crystal Form A

10 mg of compound 1 was weighed out, added to 0.1 mL of ethyl acetate, and dissolved by stirring at room temperature, and 0.4 mL of n-heptane was added. After 1 day of stirring, volatilization was performed for crystallization to give a product.

The product was identified as the crystal form A by X-ray powder diffraction.

### Example 28: Preparation of Crystal Form A

10 mg of compound 1 was weighed out, added to 0.1 mL of methyl tert-butyl ether, and dissolved by stirring at room temperature, and 0.1 mL of cyclohexane was added for precipitation. Another 0.2 mL of cyclohexane was added, and the mixture was then stirred for 1 day. After filtration, the solid was dried *in vacuo* to give a product.

The product was identified as the crystal form A by X-ray powder diffraction.

### Example 29: Preparation of Crystal Form A

10 mg of compound 1 was weighed out, added to 0.1 mL of tetrahydrofuran, and dissolved by stirring at room temperature, and 0.4 mL of cyclohexane was added. After 1 day of stirring, volatilization was performed for crystallization to give a product.

The product was identified as the crystal form A by X-ray powder diffraction.

### Example 30: Preparation of Crystal Form A

10 mg of compound 1 was weighed out, added to 0.1 mL of methyl isobutyl ketone, and dissolved by stirring at room temperature, and 0.4 mL of cyclohexane was added. After 1 day of stirring, volatilization was performed for crystallization to give a product.

The product was identified as the crystal form A by X-ray powder diffraction.

### Example 31: Preparation of Crystal Form A

10 mg of compound 1 was weighed out, added to 0.1 mL of 10% water/acetone, and dissolved by stirring at room temperature, and 0.1 mL of purified water was added for precipitation. Another 0.2 mL of purified water was added, and the mixture was then stirred for 1 day. After filtration, the solid was dried *in vacuo* to give a product.

The product was identified as the crystal form A by X-ray powder diffraction.

### Example 32: Preparation of Crystal Form A

10 mg of compound 1 was weighed out, added to 0.1 mL of isopropyl acetate, and dissolved by stirring at room temperature, and 0.4 mL of cyclohexane was added. Volatilization was performed for crystallization to give a product.

The product was identified as the crystal form A by X-ray powder diffraction.

### Example 33: Preparation of Crystal Form B

10 mg of compound 1 was weighed out, added to 0.1 mL of dimethyl sulfoxide, and completely dissolved, and 0.2 mL of water was added for crystallization. Then slurrying was performed for 1 day. After filtration, drying was performed *in vacuo* to give a solid.

According to X-ray powder diffraction analysis, the product was defined as crystal form B. Its XRPD pattern is shown in FIG. 2, and its characteristic peak positions are shown in Table 6.

Its DSC thermogram showed endothermic peaks at 81.99 °C and 173.56 °C.

Its TGA thermogram showed a weight loss of 18.35% from 30 °C to 100 °C.

**Table 6**

| Peak No. | 2θ [° or degrees] | d [Å] | Relative intensity (%) |
|---|---|---|---|
| 1 | 8.205 | 10.76735 | 83.2 |
| 2 | 9.781 | 9.03565 | 100.0 |
| 3 | 10.672 | 8.28326 | 13.5 |
| 4 | 12.870 | 6.87299 | 96.6 |
| 5 | 15.356 | 5.76538 | 25.2 |
| 6 | 15.907 | 5.56710 | 78.2 |
| 7 | 16.997 | 5.21220 | 33.1 |
| 8 | 19.448 | 4.56068 | 97.0 |
| 9 | 19.796 | 4.48122 | 94.9 |
| 10 | 20.264 | 4.37871 | 61.9 |
| 11 | 21.524 | 4.12524 | 31.7 |
| 12 | 22.416 | 3.96306 | 12.5 |
| 13 | 23.185 | 3.83333 | 59.8 |
| 14 | 24.053 | 3.69692 | 6.8 |
| 15 | 24.803 | 3.58678 | 23.7 |
| 16 | 25.118 | 3.54254 | 14.0 |
| 17 | 26.235 | 3.39414 | 20.3 |
| 18 | 26.499 | 3.36087 | 21.0 |
| 19 | 26.854 | 3.31732 | 14.5 |
| 20 | 27.136 | 3.28347 | 33.9 |
| 21 | 28.114 | 3.17142 | 29.6 |
| 23 | 29.336 | 3.04205 | 3.0 |
| 22 | 29.651 | 3.01040 | 2.6 |
| 23 | 30.080 | 2.96849 | 6.7 |
| 24 | 31.028 | 2.87989 | 8.0 |
| 25 | 31.764 | 2.81486 | 5.3 |
| 26 | 32.497 | 2.75301 | 13.7 |
| 27 | 34.183 | 2.62100 | 2.3 |
| 28 | 35.204 | 2.54728 | 8.7 |
| 29 | 36.073 | 2.48788 | 1.0 |
| 30 | 38.927 | 2.31177 | 2.9 |
| 31 | 40.207 | 2.24108 | 0.5 |
| 32 | 41.151 | 2.19182 | 2.5 |
| 33 | 42.494 | 2.12562 | 2.8 |
| 34 | 43.606 | 2.07395 | 2.0 |
| 35 | 45.221 | 2.00355 | 1.8 |
| 36 | 46.438 | 1.95384 | 2.3 |
| 37 | 47.466 | 1.91389 | 1.6 |

### Example 34: Preparation of Crystal Form C

30 mg of compound 1 was weighed out, added to 1.2 mL of 1,4-dioxane, and dissolved by stirring, and 1.8 mL of n-heptane was added. The solution was stirred for crystallization. After filtration, drying was performed *in vacuo* to give a solid.

According to X-ray powder diffraction analysis, the product was defined as crystal form C. Its XRPD pattern is shown in FIG. 3, and its characteristic peak positions are shown in Table 7.

Its DSC thermogram showed endothermic peaks at 141.90 °C and 176.81 °C.

Its TGA thermogram showed a weight loss of 1.89% from 30 °C to 85 °C and a weight loss of 5.94% from 85 °C to 160 °C.

**Table 7**

| Peak No. | 2θ [° or degrees] | d [Å] | Relative intensity (%) |
|---|---|---|---|
| 1 | 7.353 | 12.01222 | 17.8 |
| 2 | 7.753 | 11.39449 | 18.7 |
| 3 | 9.296 | 9.50591 | 100.0 |
| 4 | 14.475 | 6.11415 | 17.1 |
| 5 | 15.522 | 5.70430 | 45.9 |
| 6 | 16.248 | 5.45098 | 13.3 |
| 7 | 17.320 | 5.11583 | 15.8 |
| 8 | 18.784 | 4.72026 | 27.5 |
| 9 | 20.139 | 4.40570 | 6.0 |
| 10 | 21.083 | 4.21048 | 14.8 |
| 11 | 21.398 | 4.14925 | 31.4 |
| 12 | 22.765 | 3.90309 | 12.0 |
| 13 | 23.216 | 3.82817 | 37.4 |
| 14 | 24.347 | 3.65291 | 17.2 |
| 15 | 25.172 | 3.53497 | 10.1 |
| 16 | 25.889 | 3.43878 | 35.5 |
| 17 | 28.443 | 3.13546 | 10.7 |
| 18 | 28.896 | 3.08733 | 49.6 |
| 19 | 29.287 | 3.04701 | 13.4 |
| 20 | 30.586 | 2.92053 | 2.2 |
| 21 | 34.533 | 2.59517 | 7.7 |
| 22 | 35.403 | 2.53340 | 1.4 |
| 23 | 36.114 | 2.48515 | 1.0 |
| 24 | 38.122 | 2.35873 | 4.6 |
| 25 | 39.804 | 2.26286 | 1.8 |
| 26 | 43.640 | 2.07239 | 6.8 |
| 27 | 48.281 | 1.88349 | 3.2 |
| 28 | 48.669 | 1.86938 | 1.7 |

### Example 35: Preparation of Crystal Form D

30 mg of compound 1 was weighed out, added to 0.3 mL of tetrahydrofuran, and dissolved by stirring at room temperature, and 1.2 mL of water was added. The solution was stirred for crystallization. After filtration, drying was performed *in vacuo* to give a solid product.

According to X-ray powder diffraction analysis, the product was defined as crystal form D. Its XRPD pattern is shown in FIG. 4, and its characteristic peak positions are shown in Table 8.

Its DSC thermogram showed an endothermic peak at 176.85 °C.

Its TGA thermogram showed a weight loss of 0.34% from 30 °C to 100 °C.

**Table 8**

| Peak No. | 2θ [° or degrees] | d [Å] | Relative intensity (%) |
|---|---|---|---|
| 1 | 7.350 | 12.01838 | 17.7 |
| 2 | 10.968 | 8.06004 | 5.6 |
| 3 | 11.696 | 7.56025 | 11.3 |
| 4 | 12.084 | 7.31807 | 16.2 |
| 5 | 15.384 | 5.75490 | 100.0 |
| 6 | 16.260 | 5.44701 | 15.7 |
| 7 | 16.857 | 5.25538 | 8.0 |
| 8 | 17.855 | 4.96369 | 14.0 |
| 9 | 18.643 | 4.75572 | 32.2 |
| 10 | 21.610 | 4.10892 | 24.8 |
| 11 | 22.377 | 3.96982 | 10.3 |
| 12 | 22.768 | 3.90247 | 35.9 |
| 13 | 24.347 | 3.65294 | 61.4 |
| 14 | 25.201 | 3.53107 | 31.0 |
| 15 | 26.038 | 3.41936 | 63.8 |
| 16 | 27.012 | 3.29821 | 4.7 |
| 17 | 28.392 | 3.14096 | 2.7 |
| 18 | 29.312 | 3.04452 | 40.3 |
| 19 | 29.822 | 2.99353 | 1.2 |
| 20 | 30.610 | 2.91830 | 13.1 |
| 21 | 31.157 | 2.86831 | 3.0 |
| 22 | 36.089 | 2.48679 | 2.7 |
| 23 | 42.497 | 2.12548 | 3.1 |
| 24 | 47.526 | 1.91164 | 2.1 |
| 25 | 48.686 | 1.86876 | 2.0 |

### Example 36: Preparation of Crystal Form E

100 mg of compound 1 was weighed out, dispersed in 1 mL of acetonitrile, and slurried by stirring for 48 h. After filtration, the solid was collected and dried *in vacuo* at 45 °C to give a solid product.

According to X-ray powder diffraction analysis, the product was defined as crystal form E. Its X-ray powder diffraction data are shown in Table 9, and its X-ray powder diffraction pattern is shown in FIG. 5.

Its DSC thermogram showed endothermic peaks at 88.25 °C and 177.28 °C.

Its TGA thermogram showed that the compound had a weight loss of 1.80% from 30 °C to 115 °C and a weight loss of 5.29% from 115 °C to 230 °C.

**Table 9**

| Peak No. | 2θ value [° or degrees] | D[Å] | Relative intensity% |
|---|---|---|---|
| 1 | 11.608 | 7.61750 | 2.7 |
| 2 | 13.489 | 6.55904 | 11.3 |
| 3 | 13.753 | 6.43354 | 3.7 |
| 4 | 14.587 | 6.06769 | 5.0 |
| 5 | 14.893 | 5.94383 | 3.6 |
| 6 | 16.863 | 5.25352 | 6.8 |
| 7 | 18.000 | 4.92413 | 22.8 |
| 8 | 18.943 | 4.68101 | 4.6 |
| 9 | 19.774 | 4.48624 | 3.6 |
| 10 | 22.024 | 4.03269 | 1.3 |
| 11 | 23.279 | 3.81807 | 5.3 |
| 12 | 23.559 | 3.77322 | 17.1 |
| 13 | 24.276 | 3.66346 | 100.0 |
| 14 | 25.768 | 3.45457 | 8.3 |
| 15 | 26.108 | 3.41042 | 14.1 |
| 16 | 26.328 | 3.38234 | 40.3 |
| 17 | 27.094 | 3.28845 | 24.3 |
| 18 | 32.370 | 2.76350 | 1.0 |
| 19 | 33.412 | 2.67964 | 1.3 |
| 20 | 33.942 | 2.63903 | 1.3 |
| 21 | 37.155 | 2.41787 | 1.5 |
| 22 | 37.931 | 2.37019 | 3.7 |
| 23 | 42.766 | 2.11274 | 1.9 |

### Example 37: Preparation of Crystal Form F

10 mg of compound 1 was weighed out, added to 0.4 mL of 1,4-dioxane, and completely dissolved, and 0.4 mL of n-heptane was added. The solution was stirred for 1 day for crystallization. An additional 0.2 mL of n-heptane was added, and slurrying was performed for 3 h. After filtration, the supernatant was volatilized for crystallization to give a solid product.

According to X-ray powder diffraction analysis, the product was defined as crystal form F. Its XRPD pattern is shown in FIG. 6, and its characteristic peak positions are shown in Table 10.

**Table 10**

| Peak No. | 2θ [° or degrees] | d [Å] | Relative intensity (%) |
|---|---|---|---|
| 1 | 7.734 | 11.42250 | 20.7 |
| 2 | 9.299 | 9.50244 | 86.2 |
| 3 | 13.123 | 6.74113 | 5.6 |
| 4 | 14.439 | 6.12952 | 25.1 |
| 5 | 15.621 | 5.66819 | 100.0 |
| 6 | 16.200 | 5.46694 | 20.9 |
| 7 | 17.107 | 5.17894 | 28.3 |
| 8 | 17.314 | 5.11762 | 24.1 |
| 9 | 18.822 | 4.71098 | 16.9 |
| 10 | 20.105 | 4.41297 | 10.8 |
| 11 | 21.055 | 4.21597 | 38.6 |
| 12 | 21.395 | 4.14985 | 50.1 |
| 13 | 23.194 | 3.83189 | 39.1 |
| 14 | 23.545 | 3.77550 | 19.9 |
| 15 | 25.814 | 3.44850 | 80.4 |
| 16 | 27.027 | 3.29647 | 4.6 |
| 17 | 27.399 | 3.25256 | 10.4 |
| 18 | 28.554 | 3.12355 | 16.1 |
| 19 | 28.906 | 3.08634 | 55.8 |
| 20 | 30.539 | 2.92493 | 5.1 |
| 21 | 31.409 | 2.84582 | 1.1 |
| 22 | 34.485 | 2.59871 | 37.0 |
| 23 | 35.498 | 2.52682 | 2.1 |
| 24 | 36.771 | 2.44219 | 13.4 |
| 25 | 38.110 | 2.35944 | 4.0 |
| 26 | 38.809 | 2.31854 | 0.7 |
| 27 | 39.824 | 2.26174 | 20.2 |
| 28 | 43.544 | 2.07676 | 31.7 |
| 29 | 44.678 | 2.02663 | 5.8 |

### Test Example 2: Influencing Factors

Samples of the crystal form A were taken, spread in open containers, and left to stand under conditions of light exposure (4500 Lux), high temperatures (40 °C and 60 °C), and high humidities (RH 75% and RH 92.5%) for 30 days to study its stability.

**Table 11. The influencing factor stability of the crystal form**

| Conditions | Time (days) | Color and state | Purity% | Crystal form |
|---|---|---|---|---|
| Initial | 0 | Off-white solid | 100.0 | Crystal form A |
| Light exposure (4500 Lux) | 5 | Off-white solid | 100.0 | Did not change |
| | 10 | Off-white solid | 100.0 | Did not change |
| | 30 | Off-white solid | 99.9 | Did not change |
| 40°C | 5 | Off-white solid | 100.0 | Did not change |
| | 10 | Off-white solid | 100.0 | Did not change |
| | 30 | Off-white solid | 100.0 | Did not change |
| 60°C | 5 | Off-white solid | 100.0 | Did not change |
| | 10 | Off-white solid | 100.0 | Did not change |
| | 30 | Off-white solid | 100.0 | Did not change |
| 75% RH | 5 | Off-white solid | 100.0 | Did not change |
| | 10 | Off-white solid | 100.0 | Did not change |
| | 30 | Off-white solid | 100.0 | Did not change |
| 92.5% RH | 5 | Off-white solid | 100.0 | Did not change |
| | 10 | Off-white solid | 100.0 | Did not change |
| | 30 | Off-white solid | 100.0 | Did not change |

Conclusion: The influencing factor experiments show that: standing under conditions of light exposure, high temperatures (40 °C and 60 °C), and high humidities (75% and 92.5%) for 30 days, the free-state crystal form A exhibited both good physical stability and good chemical stability.

### Test Example 3: Long-Term Accelerated Tests

The crystal form A was left to stand under conditions of 25 °C/60% RH and 40 °C/75% RH to study its stability.

**Table 12. The long-term accelerated stability of the crystal form**

| Test conditions | Purity (%) | | | | | Crystal form |
|---|---|---|---|---|---|---|
| | Initial | 1M | 2M | 3M | 6M | Crystal form A |
| 25 °C, 60% RH | 100.0 | 100.0 | 100.0 | 100.0 | 100.0 | Did not change |
| 40 °C, 75% RH | 100.0 | 100.0 | 100.0 | 100.0 | 100.0 | Did not change |

Conclusion: The long-term accelerated experiments show that: standing under conditions of 25 °C/60% RH and 40 °C/75% RH for 6 months, the crystal form A exhibited both good physical stability and good chemical stability.

## Claims

1. A crystal form A of the compound 8-chloro-*N*-(2,2-difluorobenzo[*d*][1,3]dioxol-5-yl)quinolin-2-amine, wherein an X-ray powder diffraction pattern expressed in terms of 2*θ* angles, which are diffraction angles, of the crystal form has characteristic peaks at 7.237, 9.232, 13.702, 14.459, and 18.917, preferably at 7.237, 9.232, 13.702, 14.459, 18.917, 24.428, and 29.321, and more preferably at 7.237, 9.232, 13.702, 14.459, 18.033, 18.917, 24.428, 25.521, and 29.321; most preferably, the X-ray powder diffraction pattern expressed in terms of 2*θ* angles, which are diffraction angles, is shown in FIG. 1.

2. A crystal form B of the compound 8-chloro-*N*-(2,2-difluorobenzo[*d*][1,3]dioxol-5-yl)quinolin-2-amine, wherein an X-ray powder diffraction pattern expressed in terms of 2*θ* angles, which are diffraction angles, of the crystal form has characteristic peaks at 8.205, 9.781, 12.870, 15.907, and 19.796, preferably at 8.205, 9.781, 12.870, 15.907, 19.448, 19.796, 20.264, and 23.185, and more preferably at 8.205, 9.781, 10.672, 12.870, 15.356, 15.907, 16.997, 19.448, 19.796, 20.264, and 23.185; most preferably, the X-ray powder diffraction pattern expressed in terms of 2*θ* angles, which are diffraction angles, is shown in FIG. 2.

3. A crystal form C of the compound 8-chloro-*N*-(2,2-difluorobenzo[*d*][1,3]dioxol-5-yl)quinolin-2-amine, wherein an X-ray powder diffraction pattern expressed in terms of 2*θ* angles, which are diffraction angles, of the crystal form has characteristic peaks at 9.296, 15.522, 18.784, 23.216, and 25.889, preferably at 7.753, 9.296, 15.522, 18.784, 21.398, 23.216, and 25.889, and more preferably at 7.353, 7.753, 9.296, 14.475, 15.522, 16.248, 17.320, 18.784, 21.398, 23.216, and 25.889; most preferably, the X-ray powder diffraction pattern expressed in terms of 2*θ* angles, which are diffraction angles, is shown in FIG. 3.

4. A crystal form D of the compound 8-chloro-*N*-(2,2-difluorobenzo[*d*][1,3]dioxol-5-yl)quinolin-2-amine, wherein an X-ray powder diffraction pattern expressed in terms of 2*θ* angles, which are diffraction angles, of the crystal form has characteristic peaks at 7.350, 12.084, 15.384, 18.643, and 29.312, preferably at 7.350, 12.084, 15.384, 16.260, 17.855, 18.643, 21.610, and 29.312, and more preferably at 7.350, 12.084, 15.384, 16.260, 17.855, 18.643, 21.610, 22.768, 24.347, 25.201, 26.038, and 29.312; most preferably, the X-ray powder diffraction pattern expressed in terms of 2*θ* angles, which are diffraction angles, is shown in FIG. 4.

5. A crystal form E of the compound 8-chloro-*N*-(2,2-difluorobenzo[*d*][1,3]dioxol-5-yl)quinolin-2-amine, wherein an X-ray powder diffraction pattern expressed in terms of 2*θ* angles, which are diffraction angles, of the crystal form has characteristic peaks at 13.489, 18.000, 23.559, 24.276, and 26.328, preferably at 13.489, 16.863, 18.000, 23.559, 24.276, 26.108, 26.328, and 27.094, and more preferably at 13.489, 14.587, 16.863, 18.000, 18.943, 23.279, 23.559, 24.276, 25.768, 26.108, 26.328, and 27.094; most preferably, the X-ray powder diffraction pattern expressed in terms of 2*θ* angles, which are diffraction angles, is shown in FIG. 5.

6. A crystal form F of the compound 8-chloro-*N*-(2,2-difluorobenzo[*d*][1,3]dioxol-5-yl)quinolin-2-amine, wherein an X-ray powder diffraction pattern expressed in terms of 2*θ* angles, which are diffraction angles, of the crystal form has characteristic peaks at 9.299, 14.439, 15.621, 16.200, and 17.314, preferably at 7.734, 9.299, 14.439, 15.621, 16.200, 17.314, 21.395, and 25.814, and more preferably at 7.734, 9.299, 14.439, 15.621, 16.200, 17.314, 21.055, 21.395, 23.194, 25.814, 28.906, 34.485, and 43.544; most preferably, the X-ray powder diffraction pattern expressed in terms of 2*θ* angles, which are diffraction angles, is shown in FIG. 6.

7. The crystal form according to any one of claims 1-6, wherein the 2*θ* values have a margin of error of ±0.2.

8. A preparation method for preparing the crystal form according to any one of claims 1-7, wherein the preparation method is selected from the group consisting of any one of the following methods:
method 1:
(a) mixing the compound 8-chloro-*N*-(2,2-difluorobenzo[*d*][1,3]dioxol-5-yl)quinolin-2-amine with a solvent and stirring to dissolve or heating to dissolve, and
(b) adding a second solvent and crystallizing;
and, method 2:
(a) mixing the compound 8-chloro-*N*-(2,2-difluorobenzo[*d*][1,3]dioxol-5-yl)quinolin-2-amine with a solvent and stirring to dissolve or heating to dissolve, and
(b) crystallizing;
and, method 3:
(a) mixing the compound 8-chloro-*N*-(2,2-difluorobenzo[*d*][1,3]dioxol-5-yl)quinolin-2- amine with a solvent,
and
(b) slurrying by stirring.

9. A pharmaceutical composition, comprising the crystal form according to any one of claims 1-7 and a pharmaceutically acceptable excipient.

10. A pharmaceutical composition, wherein the pharmaceutical composition is prepared from the crystal form according to any one of claims 1-7 and a pharmaceutically acceptable excipient.

11. Use of the crystal form according to any one of claims 1-7 or the pharmaceutical composition according to claim 9 or 10 in the preparation of a medicament for treating and/or preventing a disease or condition, wherein the disease or condition is selected from the group consisting of an inflammation and a cancer; the inflammation is preferably inflammatory bowel disease, and the cancer is preferably melanoma or breast cancer.
